# EUROPEAN PATENT SPECIFICATION

(11) **EP 0 462 551 B1**
(45) Date of publication and mention of the grant of the patent: **04.10.1995**
(21) Application number: 91109937.2
(22) Date of filing: 18.06.1991
(51) Int. Cl.: C12P 21/08, C12N 5/20, G01N 33/569, G01N 33/577, A61K 39/42

(54) **HIV GP120 monoclonal antibodies**
Monoklonale Antikörper gegen HIV GP120
Anticorps monoclonaux contre la GP120 de VIH

(30) Priority: 18.06.1990 US 540025
(43) Date of publication of application: 27.12.1991
(73) Proprietor: ABBOTT LABORATORIES, Abbott Park, Illinois 60064-3500 (US)
(72) Inventor: Chin, Jade, Edison, NJ 08817 (US); Gibadlo, Mary S., Palatine, IL 60067 (US); Robey, W. Gerard, Libertyville, IL 60048 (US)
(74) Representative: Modiano, Guido, Dr.-Ing.

(56) References cited:
- WO-A-91/11528
- JOURNAL OF VIROLOGY, vol. 64, no. 6, June 1990, Baltimro, MD (US); J. HANSEN et al., pp. 2833-2840/
- AIDS, vol. 4, no. 4, April 1990, London (GB); J. MOORE et al., pp. 307-315/
- JOURNAL OF GENERAL VIROLOGY, vol. 69, 1988, Colchester (GB); R. MONTELARO et al., pp. 1711-1717/
- JOURNAL OF VIROLOGY, vol. 62, no. 11, November 1988, Baltimore, MD (US); M. SKINNER et al., pp. 4195-4200/

## Description

### Field of the Invention

The present invention relates to monoclonal antibodies capable of substantially neutralizing the activity of human immunodeficiency viruses and to hybridomas which produce such antibodies.

### Related Art

Human immunodeficiency virus (HIV) is the etiologic agent of the acquired immunodeficiency syndrome (AIDS) and related disorders such as AIDS related complex (ARC). HIV recognizes a receptor, CD4, specific to the helper/inducer subset of T lymphocytes (Dalgleish et al., Nature (1984) 312:763-67). This subset of lymphocytes is depleted in AIDS patients. HIV-induced immunosuppression compromises the AIDS patient's ability to fight off opportunistic infections such as Pneumocystic carinii pneumonia and unusual neoplasms such as Kaposi's sarcoma.

The binding of HIV to its cellular receptor, CD4, is mediated by proteins encoded by the viral envelope (env) gene. The HIV viral envelope protein is expressed as a precursor protein, gp160, that is proteolytically cleaved to generate an external envelope glycoprotein, gp120, and a transmembrane envelope glycoprotein, gp41. Gp120 is heavily glycosylated. Ratner et al. (Nature (1985) 313:277-84) identified more than 20 asparagine residues which can serve as potential N-glycosylation sites. Oligomannosidic and fucosylated, partially sialylated bi- and triantennary complex carbohydrates comprise approximately half of the mass of gp120 (Geyer et al., J. Biol. Chem. (1988) 263:11760-67). At present, the precise role of the carbohydrates of gp120 in the binding of the gp120 molecule to the CD4 receptor is poorly understood. However, there is evidence that CD4 binding requires glycosylation as described in Matthews et al., Proc. Natl. Acad. Sci. USA (1987) 84:5424-28.

Another role of the gp120 molecule relates to its capability of inducing neutralizing antibodies. For example, Robey et al. (Proc. Natl. Acad. Sci. USA (1986) 83:7023-27) reported that purified gp120 induced polyclonal neutralizing antibodies in experimental animals. In addition, murine monoclonal neutralizing antibodies to the gp120 protein have been successfully produced (Skinner et al., AIDS Res. Hum. Retroviruses (1988) 4:187-97; Matsushita et al., J. Virol. (1988) 62:2107-14). These antibodies have been shown to block the infectivity of T cells in vitro. By testing whether these monoclonal antibodies bound to nonglycosylated recombinant proteins and synthetic peptides of gp120, a primary epitope associated with these neutralizing antibodies has been localized to amino acid sequence 301-341 of the gp120 protein. A disulfide loop configured by cysteine residues 303 and 338 of gp120 appears to be essential to maintaining the conformational integrity of this epitope (Javaherian et al., Proc. Natl. Acad. Sci. USA (1989) 86:6768-72). Known gp120 neutralizing monoclonal antibodies bind to nonglycosylated gp120 recombinant proteins. Therefore, it is likely that such monoclonal antibodies recognize the polypeptide chain of the HIV gp120 protein.

Hansen et al., Journal of Virology, (June 1990) 64, no. 6, pp. 2833-2840 disclose the inhibition of in vitro HIV infectivity by anti-carbohydrate monoclonal antibodies. The virus strains involved were HTLV-III_{B} and a patient isolate. The MAbs used were known antibodies with well defined anti-carbohydrate specificity for carbohydrate structures located at the periphery of both N-linked and O-linked, or exclusively O-linked, chains of glycoproteins and also glycosphingolipids. The utilized MAbs were not derived from HIV-1. Likewise, WO-A-9 111 528, which is considered prior art under Article 54(3) and (4) EPC, discloses the inhibition of in vitro HIV infectivity (virus strains HTLV-III_{B} and a patient isolate) by anti-carbohydrate monoclonal antibodies defining carbohydrate structures found at the periphery of O-linked chains of glycoproteins. Again the utilized MAbs were not derived from HIV-1. The carbohydrate epitopes associated with the HIV envelope protein gp120 that were identified were said to not be normally expressed in the human organism.

Skinner et al. Journal of Virology, (Nov. 1988), 62, no. 11, pp. 4195-4200 disclose monoclonal antibodies which possess HTLV-III_{B} neutralizing activity but which do not prevent gp120 binding to cells which express CD4 surface antigen. The target epitope is the polypeptide chain falling within gp120 residues 307 and 330.

### SUMMARY OF THE INVENTION

The present invention provides monoclonal antibodies characterized as being specific for an epitope on a HIV glycoprotein having a molecular weight of about 120,000 daltons (gp120). Such antibodies recognize a carbohydrate portion of gp120 localized in the region from about amino acid 301 to about amino acid 358. The invention also provides hybridomas which produce such monoclonal antibodies.

### DETAILED DESCRIPTION OF THE INVENTION

The present invention provides monoclonal antibodies which are capable of substantially neutralizing the HIV virus and hybridomas which produce such monoclonal antibodies. The monoclonal antibodies neutralize diverse strains of both laboratory and clinical HIV strains. The term "neutralizing" as used herein denotes the inhibition of HIV infectivity in H9 cells in vitro.

The monoclonal antibodies of the present invention are capable of specifically binding to the external envelope glycoprotein gp120 as well as its precursor protein gp160. The monoclonal antibodies are characterized by their recognition of an epitope in the neutralizing region of the gp120 protein. The monoclonal antibodies are further characterized by their recognition of the carbohydrate portion of the major gp120 neutralizing region, localized in the region from about amino acid 301 to about amino acid 358.

Two especially preferred monoclonal antibodies of the invention are monoclonal antibodies 52-581-290 and 52-684-238. The hybridomas which produce monoclonal antibodies 52-581-290 and 52-684-238 were deposited at the American Type Culture Collection, Rockville, Maryland, on March 29, 1990, under deposit accession numbers HB10399 and HB10400, respectively.

The monoclonal antibodies of the present invention may be used for the diagnosis, prevention and treatment of AIDS. The antibodies can be employed to better characterize the gp120 antigen, which has been shown to bind the CD4 receptor of susceptible T4 cells as an initial event in the infection process. It is also possible to use the monoclonal antibodies of the present invention to inhibit the growth of the virus in human hosts or to inhibit further infection by preventing infection of uninfected T4 cells, due to their ability to inhibit HIV infectivity in vitro.

The following Methods and Examples detail the preparation and characterization of monoclonal antibodies of the present invention.

### METHODS

### Purification of native gp120

The HIV gp120 antigen was purified according to the method of Robey et al., Proc. Natl. Acad. Sci. USA (1986) 83:7023-27. Briefly, H9 cells infected with HTLV-IIIB (HTLV-III prototype strain obtained from R. Gallo, National Institutes of Health) were resuspended in phosphate buffered saline (PBS) containing 0.5% Triton® X-100 and 1 mM phenylmethylsulfonyl fluoride (PMSF). Cells were resuspended at a ratio of from about 6 mL to about 10 mL of buffer to 1 g cell pellet. The resuspended cells were centrifuged at 500 x g for 10 minutes. The supernatant was frozen overnight at -20°C, thawed and then refrozen. The twice-thawed supernatant was centrifuged at 9000 rpm (JA10 rotor) for 20 minutes. The supernatant was centrifuged through a continuous flow, high speed rotor (CF32) at 30,000 rpm at a flow rate of from about 2 L/hour to about 3 L/hour. The recovered supernatant was adjusted to 1.0 M KCl and 0.01 M Tris(hydroxymethyl)aminomethane (Tris), pH 8.5. The supernatant was mixed with a gp41 monoclonal antibody resin, and the column flow-through fraction (i.e., that fraction which did not bind to the gp41 antibody) was added to a polyclonal AIDS antibody resin, which was selected to contain primarily antibodies to env gene products. The mixture was incubated overnight at room temperature, while rotated on a roller mill. The mixture was subsequently poured into a column. The column was eluted with 4 M MgCl₂. The eluted gp120 was dialyzed against water, storing at about 4°C, changing water 2 times daily, until a precipitate formed (about 2 days). The dialyzed gp120 was clarified by centrifuging at 12,000 rpm for 30 minutes. The supernatant was frozen with liquid nitrogen and lyophilized overnight. This step was repeated if any turbidity remained when the lyophilized gp120 was resolubilized in water. The lyophilized gp120 was resolubilized in water, and the mixture was clarified if turbid.

### Enzyme immunoassay (EIA)

Polystyrene 96-well microtiter plates were coated overnight at room temperature with 100 µl per well of a 1 µg/mL gp120 antigen solution in PBS, pH 7.4. Any remaining binding sites on the polystyrene wells were blocked with 3% bovine serum albumin (BSA; Armour Pharmaceutical Co., Kankakee, IL) in PBS for about 30 minutes at room temperature. Plates were washed three times with water. Dilutions of mouse sera, made in Iscove's modified Dulbecco's medium (IMDM; Gibco, Grand Island, NY) containing 10% fetal bovine serum (FBS; Hyclone, Logan, UT) or monoclonal antibody tissue culture supernatants were incubated for about 2 hours at room temperature in the wells, and the wells were washed three times with water. Monoclonal antibody binding was detected using goat anti-mouse IgG+M-horseradish peroxidase (HRPO) (Kirkegaard-Perry Laboratories (KPL), Gaithersburg, MD) diluted at a concentration of 1:1000 in PBS. O-phenylenediamine substrate (OPD; Abbott Laboratories, Abbott Park, IL) was used as the chromogen and plates were read at 490 nanometers (nm).

### Western blot

HIV-1 proteins were electrophoresed by sodium dodecyl sulfatepolyacrylamide gel electrophoresis (SDS-PAGE) and then transferred to nitrocellulose, according to the manufacturers' instructions (Schleicher & Schuell, Keene, NH; Hoefer Scientific Instruments Co., San Francisco, CA; Bio-Rad, Richmond, CA). The nitrocellulose strips were blocked with 1% bovine hemoglobin (Sigma Chemical Co., St. Louis, MO) and 0.5% Tween® 20 (Fisher Scientific, Pittsburgh, PA) in PBS for 30 minutes at room temperature, then the strips were incubated with tissue culture supernatants or dilutions of the purified antibodies made in the blocking solution. The strips were washed with PBS after the incubation period. Antigen visualization was accomplished with goat anti-mouse IgG+M-HRPO and 4-chloro-1-naphthol as the chromogenic substrate.

### Radioimmunoprecititation

The radioimmunoprecipitation (RIPA) procedure was carried out by an established protocol (Sun et al., J. Virol. (1989) 63:3579-85). Briefly, H9 cells (infected with the HIV isolates IIIB, RF, MN, AL, Z34, Z84 and AC) were metabolically labeled for 4 hours with [³⁵S]cysteine and [³⁵S]methionine (100 µCi/mL; ICN Pharmaceuticals Inc., Irvine, CA) and suspended in a RIPA lysing buffer (50 mM Tris hydrochloride, pH 8.0, 150 mM NaCl, 1% Triton® X-100, 1% sodium deoxycholate, 0.1% sodium dodecyl sulfate (SDS) and 1 mM PMSF). Lysates were precleared with protein A-Sepharose (Sigma) bound to rabbit antiserum to mouse kappa light chain (Cappel Laboratories, West Chester, PA) (k-PAS) for 3 hours at room temperature. RIPA was performed by adding 3 µg of purified mouse monoclonal antibody and 0.2 mL of a 10% suspension of k-PAS to 200 µl of labeled and clarified lysate. The samples were incubated at 4°C for 18 hours, and the beads were washed with the RIPA lysing buffer. The pellets were suspended in electrophoresis sample buffer and boiled for 3 minutes. Proteins were analyzed by SDS-PAGE, followed by autoradiography.

### Peptide synthesis

Peptide sequences of the HIV-1 gp120 envelope protein were synthesized according to the general procedure described by Barary and Merrifield (Gross & Meinehofer, eds., The Peptides, vol. 2 [Academic Press, New York, 1980]). The synthesized sequences were amino acids 37-59, 68-85, 272-323, 329-384, 462-518 and 491-518, numbered according to Ratner et al. (Nature (1985) 313:277-84).

The peptide corresponding to sequence 272-323 of the HIV-1 gp120 protein was assembled on a resin support by stepwise solid phase synthesis, starting with the carboxyl terminal residue. A BOC-L-Ala-OCH₂-Pam resin was transferred to a reaction vessel of an Applied Biosystems synthesizer, Model 430A. Protected amino acids were double coupled in a stepwise manner to the resin support by preformed symmetric anhydride chemistry, except for the addition of arginine, asparagine, and glutamine, wherein the DCC/HOBT protocol described by Konig and Geiger was used (Chem. Ber. (1970) 103:788-798). All a-aminoterminal residues were protected by t-butyloxycarbonyl (t-BOC) linkage, and side chains of various amino acid residues were protected by the following groups: Glu, OBzl; Arg, Tos; Ser, Bzl; Thr, Bzl; Asp, OBzl; Lys, 2-Cl-Z; and Cys, 4MeBzl.

Three hundred milligrams (300 mg) of the protected peptide-resin were allowed to swell in methylene chloride (CH₂Cl₂) for 5 minutes. The N^{a}-BOC protecting groups were removed using 60% trifluoroacetic acid (TFA/CH₂Cl₂), CH₂Cl₂ washes, 10% N,N-diisopropylethylamine (DIEA/CH₂Cl₂) neutralization, and final CH₂Cl₂ washes. The resin was dried in vacuo. The peptide-resin thus obtained was treated with 9 mL of anhydrous hydrofluoric acid (HF), to which 0.5 mL p-cresol and 0.5 g p-thiocresol had been added (HF "cocktail"), for 60 minutes at 0°C. The HF was distilled off in vacuo at 0°C. The cleaved, free peptide and resin were washed 3 times with 15 mL aliquots of diethyl ether. The peptide was then extracted six times with 10 mL aliquots of 40% aqueous acetic acid. The combined aqueous extracts were lyophilized to provide the crude peptide for purification.

The crude peptide was purified by reverse-phase high performance liquid chromatography (HPLC) on C4 columns, employing gradients of 0.1% TFA/water (A) and 100% acetonitrile (B) as the solvent systems at a flow rate of l mL/min for the analytical column (Vydac-214-TP54, Vydac Separation Group, Hesperia, California) or 12 mL/min for the semipreparative one (Vydac-214-TP1022). The gradient was started with 17% B. After 3 minutes, the gradient was linearly increased to 60% B during 20 minutes, then brought back to 17% B in 2 minutes.

The presence of peptide was monitored at 225 nm and 280 nm. The composition of the peptide was confirmed by hydrolysis in 6N hydrochloric acid/0.3% phenol at 150°C for 2 hours in vacuo, and subsequently analyzed on a Beckman 6300 amino acid analyzer.

Similarly, the other gp120 peptides were assembled as described above. Additional amino acid side chains were protected by the groups Tyr, 2-Br-Z; and His, Tos. The amino acids tryptophan and methionine were used without any side chain protecting groups. After incorporation of tryptophan, indole was added at a concentration of 1% (w/v) to trifluoroacetic acid for removal of all subsequent N^{a}-protecting (BOC) groups. After incorporation of methionine, ethanedithiol was also added to trifluoroacetic acid at a concentration of 0.25% (v/v). The HF cocktail for peptides 37-59 and 491-518 comprised 9 mL HF and 1 mL p-cresol. The HF cocktail for peptide 61-78 comprised 9 mL HF, 0.5 mL p-cresol and 0.5 g p-thiocresol. The HF cocktail for peptides 322-377 and 462-518 comprised 10 mL HF, 1 mL p-cresol and 1 mL dimethylsulfide. The HF cocktail for peptide 322-377 also contained 0.2 g p-thiocresol.

### EXAMPLE 1

### Immunization of Animals and Fusion

BALB/c mice were immunized subcutaneously and intraperitoneally with 50 µg of the previously described viral gp120 in RIBI's adjuvant (RIBI Immunochem Research, Inc., Hamilton, MT) on weeks 1, 4, 5 and 7. Responding animals, assessed by the enzyme immunoassay (EIA) method described below, were boosted intravenously with 10 µg of the gp120 antigen in PBS three days prior to fusion. The mice were sacrificed, and the spleen cells were isolated.

Fusion was accomplished according to known conventional methods using SP2/O cells as the myeloma partner (G. Kohler and C. Milstein, Nature (1975) 256:495-497; J. Goding, Monoclonal Antibodies: Principles and Practice [New York: Academic Press, 1983]). Hybrids were selected by supplementing the growth medium with hypozanthine, aminopterin, and thymidine. Hybrids were screened seven to ten days post-fusion by EIA against the immunizing antigen and confirmed by western blot, as described hereinbelow. The hybrids were cloned by limiting dilution and ascites generated from the clones of interest in BALB/c mice, according to standard methods. (See generally, Goding, supra.) Antibodies were purified from the ascites fluid by Protein A-Sepharose column chromatography as described by Ey et al., Immunochem. (1978) 15:429-36.

Hybrids cloned from the fusion experiments were screened for reactivity to gp120 by the EIA method previously described, and those hybrids that reacted positively were expanded. Two monoclonal antibodies from the same fusion, designated 52-581-290 and 52-684-238, gave strong positive reactions.

### EXAMPLE 2

### Epitope Identification

Upon further testing, monoclonal antibodies 52-581-290 and 52-684-238 specifically reacted with gp120 of the immunizing strain (HTLV-IIIB) by the Western blot methodology described previously. The antibodies were also examined by RIPA, using metabolically labeled HIV-1 strains IIIB-, RF-, MN-, AL-, Z34-, Z84-, and AC-infected H9 cells. Each monoclonal antibody specifically precipitated both gp120 and gp160 of the immunizing strain IIIB. However, the two antibodies differed with respect to their reactivity with other strains of HIV-1.

**Table 1**

| Mab | IIIB | RF | MN | AL | Z34 | Z84 | AC |
|---|---|---|---|---|---|---|---|
| 52-581-290 | + | + | + | + | - | - | - |
| 52-684-238 | + | - | - | - | - | - | - |
| "+" indicates reactivity with gp120 and gp160 | | | | | | | |

As illustrated in Table 1, whereas antibody 52-581-290 crossreacted with HIV strains RF, MN and AL, antibody 52-684-238 only reacted with the immunizing strain.

Furthermore, neither monoclonal antibody 52-581-290 nor monoclonal antibody 52-684-238 reacted with the recombinant proteins pE3, pB1 or penv 9 (received from J. Ghrayeb, Centocor; S. Putney, Repligen; and S. Petteway, DuPont; respectively). The recombinant proteins pE3, pB1 and penv9 were expressed in bacteria and, therefore, were expressed as nonglycosylated proteins. The proteins extend over 84% of the envelope. PB1 is a fragment from the carboxyl-terminal half of gp120 which contains the neutralizing epitope. This unreactivity suggests that the epitopes recognized by monoclonal antibody 52-581-290 and monoclonal antibody 52-684-238 are either conformation-dependent or substantially comprise a carbohydrate residue of the gp120 protein.

### IgG isotyping

The isotype of monoclonal antibodies 52-581-290 and 52-684-238 were determined to be IgG2a and IgG1, respectively. Briefly, microtiter plates were coated with goat anti-mouse IgG + M immunoglobulin (KPL) and blocked with 3% BSA. Culture fluid samples were added to the wells, and the plates were incubated at room temperature for 1 hour. The plates were washed, labeled goat anti-mouse isotype conjugates (Southern Biotech, Birmingham, AL) were added, and the plates were incubated at room temperature for 30 minutes. The excess conjugate was removed by washing, then OPD was added. The amount of goat anti-mouse isotype bound to the mouse immunoglobulin was proportional to the absorbance measured at 490 nm.

### Peptide inhibition assays

Synthetic peptides were synthesized as previously described for HIV-1 amino acid sequences 37-59, 68-85, 272-323, 329-384, 462-518 and 491-518 (numbered according to Ratner et al., Nature (1985) 313:277-84). These peptides were employed in competition assays according to the procedure previously described by substituting serial dilutions of the peptides in place of the unlabeled antibody. Fifty microliters of either monoclonal antibody 52-581-290 or 52-684-238, or control antibodies 52-445-22 (commercially available from Abbott Laboratories, Abbott Park, IL as list no. 3A18-59) or 44-322-51 were added at a dilution equal to that which gave a value of 50% of the maximum absorbance value in a direct EIA assay. The peptides and monoclonal antibodies were added to the wells at the same time. HRPO-labeled goat anti-mouse conjugate (KPL) was employed to detect the immune complexes so formed.

**Table 2**

| Antibody | Amino Acid Sequence | | | | | |
|---|---|---|---|---|---|---|
| | 37-59 | 68-85 | 272-323 | 329-384 | 462-518 | 491-518 |
| 52-581-290 | - | - | - | - | - | - |
| 52-684-238 | - | - | - | - | - | - |
| 52-445-22 | - | - | + | - | - | - |
| 44-322-151 | - | - | + | - | - | - |

As shown in Table 2, monoclonal antibodies 52-581-290 and 52-684-238 did not recognize peptides 272-323 and 329-384, covering the neutralizing region, whereas both monoclonal antibodies 52-445-22 and 44-322-151 recognized peptide 272-323. This data suggests that if monoclonal antibodies 52-581-290 and 52-684-238 are neutralizing antibodies, they do not recognize the peptide backbone of the neutralizing region.

### Antibody competitive binding studies

Abbott and commercially available (DuPont, Wilmington, DE) anti-gp120 monoclonal antibodies were employed in a competitive binding assay with labeled monoclonal antibodies of the invention to determine their reactivity. The ability of the unlabeled antibody to compete with the binding of the labeled antibody was taken as evidence of their ability to recognize identical or adjacent epitopes. Monoclonal antibody 52-445-22 is known to react with synthetic peptide 272-323 (see above) and does not recognize the carbohydrate portion. Monoclonal antibody 44-322-151 is also known to react with synthetic peptide 272-323, but was found to recognize a distinct epitope from 52-445-22 because the two monoclonal antibodies do not compete with each other. The DuPont 9284 antibody is characterized as recognizing an epitope in the region of amino acid residues 307-319. It was determined that this antibody recognized the polypeptide backbone of gp120 (Skinner et al., AIDS Res. Hum. Retroviruses (1988) 4:187-97).

Briefly, monoclonal antibodies 52-581-290 and 52-684-238 were labeled with NHS-biotin (Pierce Chemical Co., Rockford, IL) according to the manufacturer's instructions. Microtiter wells were coated with gp120 antigen as previously described. First, serial dilutions of the unlabeled antibody were pre-incubated in the wells for 15 minutes, followed by the addition of a fixed amount of biotinylated antibody (the dilution in a direct EIA assay of the biotinylated antibody alone which gave a value of 50% of the maximum absorbance value). Plates were washed three times with water. Diluted streptavidin-HRPO (Zymed, South San Francisco, CA) was added to the wells, and the wells were incubated for 30 minutes. The plates were washed again, and OPD was added. After color development, the reaction was quenched with 1 N sulfuric acid, and the absorbance was read at 490 nm.

**Table 3**

| Unlabeled Antibody | 52-581-290* | 52-684-238* |
|---|---|---|
| 52-581-290 | + | + |
| 52-684-238 | + | + |
| 52-445-22 | - | - |
| 44-322-151 | - | - |
| 9284 (DuPont) | - | - |

| | | |
|---|---|---|
| *biotinylated antibody | | |

As shown in Table 3, the monoclonal antibodies 52-581-290 and 52-684-238 only competed with each other. Neither of the antibodies competed with any of the other antibodies, including those antibodies which map to the peptide backbone of the neutralizing region (52-445-22, 44-322-151 and 9284). These results indicate that the two monoclonals recognized an epitope within the same region on the gp120 antigen.

### V8 proteolysis of gp120

The purified gp120 previously described was digested with a glutamate-specific protease from Staphylococcus aureus V8 according to the published method of Nygren et al., Proc. Natl. Acad. Sci. USA (1988) 85:6543-46. After disulfide bond reduction, fragments with approximate molecular masses of 95, 60, 50, and 25 kilodaltons (kDa) are produced. Tests for binding to CD4-positive cells showed that only two fragments, the 95 and 25-kDa peptides, were observed in cleavage products that retain the selective binding capacity of gp120. Positions given below indicate the amino acid residues in gp120, including the signal peptide. (Ratner et al., Nature (1985) 313:277-84). Based on Nygren's provisional map of the 95, 50 and 25 kDa fragments within gp120, it is possible to position the 60 kDa fragment as a larger fragment of the 50 kDa protein, cleaved at amino acid residues 179 and 358.

After gp120 was digested with the glutamate-specific V8 protease both monoclonal antibodies 52-581-290 and 52-684-238 reacted with the 95 kDa and 60 kDa fragments of the cleaved gp120, but not with the 50 kDa fragment. This data indicated that both monoclonal antibodies bind to the gp120 protein in the region of amino acid residues 301-358.

### Endoglycosidase F and H treatment of gp120

In order to determine whether monoclonal antibodies 52-581-290 and 52-684-238 recognized the carbohydrate portion of the gp120 antigen, specific endoglycosidases were employed to selectively strip the gp120 antigen of its glycans. Native gp120 was treated with recombinant endoglycosidase H derived from Streptomyces lividans (Calbiochem, La Jolla, CA), which specifically cleaves N-linked oligosaccharides. It was used at a ratio of 10⁻³ units (U) of enzyme per 100 nanograms (ng) of gp120 antigen in 0.1 M acetate buffer, pH 5.8, for 30 minutes at 37°C. The digested gp120 antigen was electrophoresed and transferred to nitrocellulose.

Similarly, gp120 antigen was treated with endoglycosidase F (Boehringer-Mannheim, Indianapolis, IN), which removes the complex carbohydrates from the gp120 molecule. Endoglycosidase F was employed at a concentration of 0.1 U per 100 ng of gp120 in a 1% SDS buffer for 24 hours at 37°C.

Following the methods previously disclosed, gp120 was treated with endoglycosidase H, which removed approximately 60% of the asparaginelinked carbohydrates from the gp120 molecule, yielding a glycoprotein of approximately 95 kDa. The gp120 antigen was also treated with endoglycosidase F, which substantially removed the complex carbohydrates associated with the gp120 molecule. Thus, the apparent molecular weight decreased to 58-60 kDa.

Monoclonal antibody 52-581-290 reacted with the endoglycosidase H-treated gp120, but did not react with the endoglycosidase F-treated gp120. This suggests that the epitope recognized by monoclonal antibody 52-581-290 may be directed exclusively to the carbohydrate portion of the gp120 antigen. On the other hand, monoclonal antibody 52-684-238 reacted with both the endoglycosidase H-treated gp120 and the endoglycosidase F-treated gp120. This suggests that the epitope recognized by monoclonal antibody 52-684-238 may be directed to a region that is a combination of the carbohydrate and peptide backbone.

### EXAMPLE 3

### Lectin Competitive Binding Studies

The monoclonal antibodies 52-581-290 and 52-684-238 were competed against the lectins Triticum vulgaris (wheat germ), Tetragonolobus purpureas (asparagus pea), Glycine max (soybean) and Canavalia ensiformis (jack bean) Concanavalin A. Microtiter wells were coated with the previously described gp120 antigen, as described in the Methods. Fab fragments of the monoclonal antibodies 52-581-290 and 52-684-238 were generated according to the manufacturer's directions (Pierce). The Fab fragments were separated from the cleaved Fc fragments by Protein A chromatography. Biotin-labeled lectins (Sigma) were competed against Fab monoclonal antibodies for binding to the target gp120 antigen.

Briefly, the Fab fragments were serially diluted in PBS containing 0.2% FBS at a concentration from 100 µg/ml to about 0.4 µg/ml, while the lectin concentration remained constant (12.5 µg/ml for T. vulgaris and Con A, 50 µg/ml for T. purpureas and Glycine max). The Fab fragment dilutions were incubated in the wells for 15 minutes, the labeled lectins were added to the wells, and the wells were incubated for 30 minutes. The microtiter plates were washed three times with water. Diluted streptavidin-HRPO was added to the wells, and color development proceeded as described previously. Competition by the Fab fragments for lectin binding sites was defined as at least a 50% decrease in absorbance at 490 nanometers (nm) as compared with the labeled lectin reacted alone.

**Table 4**

| Lectin (Specificity) | 52-581-290 | 52-684-238 | Control |
|---|---|---|---|
| T. vulgaris (D-glcNAc)₂ | 0.4 | 0.4 | NC |
| T. purpureas (a-L-fuc) | 6.25 | 6.25 | NC |
| Glycine max (D-galNAc) | NC | NC | NC |
| Con A (a-D-man) | NC | NC | NC |
| NC=No competition. Competition is defined as the lowest concentration of monoclonal antibody (µg/ml) at which absorbance is decreased by 50%. Contol antibody is 52-445-22. | | | |

As seen in Table 4, both monoclonal antibodies 52-581-290 and 52-684-238 competed with the lectins of T. vulgaris and T. purpureas, while control antibody 52-445-22 did not compete. Neither monoclonal antibody competed with the lectins of glycine max and Concanavalin A. This data suggests that the two monoclonal antibodies recognize a part of the carbohydrate side chain that is proximal to the peptide backbone of the gp120 protein.

Furthermore, treating the gp120 with sodium periodate (NalO₄) and sodium borohydride (NaBH₄) in 2 cycles of the oxidation/reduction procedure, which reduced the terminal sialic acid sugars to their corresponding glycols, did not affect the reactivity of either monoclonal antibody 52-581-290 or 52-684-238 with the reduced gp120, as determined by Western blot. Briefly, 10 µg of gp120 were incubated with 10 µl of 0.12 M NalO₄ at room temperature for 1 hour. Twenty microliters of 0.1% NaBH₄ were added, and the mixture was incubated for an additional 1 hour. The cycle was repeated using 40 µl and 80 µl of NalO₄ and NaBH₄, respectively. Each of the monoclonal antibodies 52-581-290 and 52-684-238 specifically reacted with the reduced gp120. This data suggests that the two monoclonal antibodies do not require the sialic acid portion of the carbohydrate moiety of the gp120 in order to recognize the epitope.

### EXAMPLE 4

### Neutralization Assays

Neutralization assays were conducted according to the methods disclosed by Ho et al., Science (1988) 239:1021-23; J. Virol. (1987) 61:2024-28. Briefly, 100 µl of virus inoculum (50 50% tissue culture infective doses) was incubated with 100 µl of test antibodies of different concentrations for 1 hour at 37°C. The virus-antibody mixture was then used to infect 0.75 x 10⁶ H9 cells in 1.5 mL of RPMI 1640 medium supplemented with 15% fetal bovine serum, 10 mM HEPES (N-2-hydroxyethylpiperazine-N'-2-ethanesulfonic acid), 250 U of penicillin per mL, 250 µg of streptomycin per mL, and 2 mM L-glutamine. After 7-10 days in culture, cell-free culture supernatants were collected for assay of HIV p24 antigen by EIA (Abbott HTLV-III Antigen EIA, Abbott Laboratories).

A particular strain of HIV was considered to be neutralized by a monoclonal antibody at the concentration at which expression of HIV p24 antigen was inhibited 50% compared to control infected H9 cells (ID₅₀).

**Table 5**

| Viral Isolate | ID₅₀(µg) | |
|---|---|---|
| | 52-581-290 | 52-684-238 |
| Laboratory Strains | | |
| IIIB | 0.8 | 0.4 |
| RF | 0.7 | 2.0 |
| Z84 | 0.15 | 0.05 |
| AL | >10 | >10 |

| Clinical Strains | | |
|---|---|---|
| T.B. | 0.3 | >10 |
| L.L. | >10 | >10 |
| A.C. | 0.04 | 0.09 |
| L.S. | 0.04 | 0.03 |
| C.C. | >10 | 0.2 |
| R.P. | 0.03 | 0.07 |

As shown in Table 5, monoclonal antibodies 52-581-290 and 52-684-238 neutralized a wide variety of HIV isolates, including both laboratory and clinical strains. Those strains that still exhibited p24 expression greater than 50% of control H9 cells at 10 µg of monoclonal antibody were not considered to be neutralized.

## Claims (Claims for the following Contracting State(s): AT, BE, CH, LI, DE, DK, FR, GB, GR, IT, NL, SE)

1. A monoclonal antibody which specifically binds to an epitope on a Human Immunodeficiency Virus (HIV) glycoprotein having a molecular weight of about 120,000 daltons (gp120), characterized in that said antibody recognizes a carbohydrate portion of gp120 localized in the region from about amino acid 301 to about amino acid 358.

2. The monoclonal antibody according to claim 1 having the further characteristic of being capable of substantially neutralizing HIV.

3. A monoclonal antibody having the immunological binding characteristics of the monoclonal antibody produced by a hybridoma cell line on deposit with the American Type Culture collection, ATCC No. HB10399.

4. A monoclonal antibody having the immunological binding characteristics of the monoclonal antibody produced by a hybridoma cell line on deposit with the American Type Culture collection, ATCC No. HB10400.

5. A hybridoma capable of producing a monoclonal antibody that recognizes the carbohydrate portion of HIV gp120 wherein said monoclonal antibody binds to a carbohydrate portion localized in the region from about amino acid 301 to about amino acid 358.

6. A hybridoma cell line ATCC No. HB10399 producing antibody to the carbohydrate portion of HIV gp120.

7. A hybridoma cell line ATCC No. HB10400 producing antibody to the carbohydrate portion of HIV gp120.

8. Use of a monoclonal antibody as defined in any one of claims 1-4 in immunoassays for detecting the presence of HIV.

9. Use of a hybridoma as defined in any one of claims 5-7 for producing a monoclonal antibody.

10. Use of a monoclonal antibody as defined in any one of claims 1-4 for manufacturing a medicament for the prevention and treatment of AIDS.

## Claims (Claims for the following Contracting State(s): ES)

1. Use of a monoclonal antibody which specifically binds to an epitope on a Human Immunodeficiency Virus (HIV) glycoprotein having a molecular weight of about 120,000 daltons (gp120), characterized in that said antibody recognizes a carbohydrate portion of gp120 localized in the region from about amino acid 301 to about amino acid 358, in immunoassays for detecting the presence of HIV.

2. Use according to Claim 1 wherein the monoclonal antibody has the further characteristic of being capable of substantially neutralizing HIV.

3. Use of a monoclonal antibody having the immunological binding characteristics of the monoclonal antibody produced by a hybridoma cell line on deposit with the American Type Culture collection, ATCC No. HB10399, in immunoassays for detecting the presence of HIV.

4. Use of a monoclonal antibody having the immunological binding characteristics of the monoclonal antibody produced by a hybridoma cell line on deposit with the American Type Culture collection, ATCC No. HB10400, in immunoassays for detecting the presence of HIV.

5. Use of a hybridoma capable of producing a monoclonal antibody that recognizes the carbohydrate portion of HIV gp120 wherein said monoclonal antibody binds to a carbohydrate portion localized in the region from about amino acid 301 to about amino acid 358 for producing a monoclonal antibody.

6. Use of a hybridoma cell line ATCC No. HB10399 for producing antibody to the carbohydrate portion of HIV gp120.

7. Use of a hybridoma cell line ATCC No. HB10400 for producing antibody to the carbohydrate portion of HIV gp120.

8. Process for producing a hybridoma capable of producing a monoclonal antibody that recognizes the carbohydrate portion of HIV gp120 wherein said monoclonal antibody binds to a carbohydrate portion localized in the region from about amino acid 301 to about amino acid 358, said process comprising the steps of
(a) immunizing nonhuman animals with HIV glycoprotein gp120;
(b) screening said animals for the presence of antibody to gp120;
(c) isolating spleen cells from responding animals;
(d) fusing said spleen cells with myeloma cells;
(e) screening selected hybrids for the production of antibodies which bind to the immunizing antigen;
(f) cloning hybrids from the fusion experiments;
(g) screening the cloned hybrids for reactivity to gp120;
(h) expanding those hybrids that reacted positively;
(i) screening the hybrids for those which produce antibodies which bind to a carbohydrate portion localized within the amino acid region from about amino acid 301 to about amino acid 358.

9. Process according to Claim 8 wherein said nonhuman animals are BALB/c mice and said myeloma cells are SP2/O cells.

10. Process according to Claim 9 wherein said antibodies have the immunological binding characteristics of the monoclonal antibody produced by a hybridoma cell line on deposit with the American Type Culture collection, ATCC No. HB10399.

11. Process according to Claim 9 wherein said antibodies have the immunological binding characteristics of the monoclonal antibody produced by a hybridoma cell line on deposit with the American Type Culture collection, ATCC No. HB10400.

## Patentansprüche (Patentansprüche für folgende(n) Vertragsstaat(en): AT, BE, CH, LI, DE, DK, FR, GB, GR, IT, NL, SE)

1. Monoklonaler Antikörper, der spezifisch an ein Epitop auf einem humanen Immunschwäche-Virus(HIV)-Glycoprotein mit einem Molekulargewicht von etwa 120 000 Dalton (gp120) bindet, dadurch gekennzeichnet, daß der Antikörper einen Kohlenhydratanteil von gp120 erkennt, der sich im Bereich von etwa Aminosäure 301 bis etwa Aminosäure 358 befindet.

2. Monoklonaler Antikörper nach Anspruch 1, der desweiteren die Eigenschaft aufweist, HIV im wesentlichen zu neutralisieren.

3. Monoklonaler Antikörper, der die immunologischen Bindungseigenschaften des monoklonalen Antikörpers aufweist, der von einer Hybridomzellinie gebildet wird, die bei der American Type Culture Collection unter ATCC No. HB10399 hinterlegt ist.

4. Monoklonaler Antikörper der die immunologischen Bindungseigenschaften des monoklonalen Antikörpers aufweist, der von der Hybridomzellinie gebildet wird, die bei der American Type Culture Collection unter ATCC No. HB10400 hinterlegt ist.

5. Hybridom, das in der Lage ist, einen monoklonalen Antikörper zu bilden, der den Kohlenhydratanteil von HIV-gp120 erkennt, wobei der monoklonale Antikörper an einen Kohlenhydratanteil bindet, der sich im Bereich von etwa Aminosäure 301 bis etwa Aminosäure 358 befindet.

6. Hybridomzellinie ATCC No. HB10399, die Antikörper gegen den Kohlenhydratanteil von HIV-gp120 bildet.

7. Hybridomzellinie ATCC No. HB10400, die Antikörper gegen den Kohlenhydratanteil von HIV-gp120 bildet.

8. Verwendung eines monoklonalen Antikörpers nach einem der Ansprüche 1-4 in Immunoassays zum Nachweis der Anwesenheit von HIV.

9. Verwendung eines Hybridoms nach einem der Ansprüche 5-7 zur Bildung eines monoklonalen Antikörpers.

10. Verwendung eines monoklonalen Antikörpers nach einem der Ansprüche 1-4 für die Herstellung eines Medikaments zur Vorsorge und Behandlung von AIDS.

## Patentansprüche (Patentansprüche für folgende(n) Vertragsstaat(en): ES)

1. Verwendung eines monoklonalen Antikörpers, der spezifisch an ein Epitop auf einem humanen Immunschwäche-Virus(HIV)-Glycoprotein mit einem Molekulargewicht von etwa 120 000 Dalton (gp120) bindet, dadurch gekennzeichnet daß der Antikörper einen Kohlenhydratabschnitt von gp120 erkennt, der sich im Bereich von etwa Aminosäure 301 bis etwa Aminosäure 358 befindet, in Immunoassays zum Nachweis der Anwesenheit von HIV.

2. Verwendung nach Anspruch 1, wobei der monoklonale Antikörper desweiteren die Fähigkeit aufweist, HIV im wesentlichen zu neutralisieren.

3. Verwendung eines monoklonalen Antikörpers mit den immunologischen Bindungseigenschaften des monoklonalen Antikörpers, der von einer Hybridomzellinie gebildet wird, die bei der American Type Culture Collection unter ATCC No. HB10399 hinterlegt ist, in Immunoassays zum Nachweis der Anwesenheit von HIV.

4. Verwendung eines monoklonalen Antikörpers mit den immunologischen Bindungseigenschaften des monoklonalen Antikörpers, der von einer Hybridomzellinie gebildet wird, die bei der American Type Culture Collection unter ATCC No. HB10400 hinterlegt ist, in Immunoassays zum Nachweis der Anwesenheit von HIV.

5. Verwendung eines Hybridoms, das in der Lage ist, einen monoklonalen Antikörper zu bilden, der den Kohlenhydratanteil von HIV-gp120 erkennt, wobei dieser monoklonale Antikörper an einen Kohlenhydratanteil im Bereich von etwa Aminosäure 301 bis etwa Aminosäure 358 bindet, in Immunoassays zum Nachweis der Anwesenheit von HIV.

6. Verwendung einer Hybridomzellinie ATCC No. HB10399 zur Bildung von Antikörper gegen den Kohlenhydratanteil von HIV-gp120.

7. Verwendung einer Hybridomzellinie ATCC No. HB10400 zur Bildung von Antikörper gegen den Kohlenhydratanteil von HIV-gp120.

8. Verfahren zur Bildung eines Hybridoms, das in der Lage ist, einen monoklonalen Antikörper zu bilden, der den Kohlenhydratanteil von HIV-gp120 erkennt, wobei der monoklonale Antikörper an einen Kohlenhydratanteil im Bereich von etwa Aminosäure 301 bis etwa Aminosäure 358 bindet, wobei das Verfahren folgende Schritte umfaßt:
(a) Immunisierung von nichthumanen Tieren mit HIV-Glycoprotein gp120,
(b) Screening dieser Tiere auf die Anwesenheit von Antikörper gegen gp120,
(c) Isolierung der Milzzellen ansprechender Tiere,
(d) Fusion der Milzzellen mit Myelomzellen,
(e) Screening selektierter Hybride auf die Bildung von Antikörpern, die an das immunisierende Antigen binden,
(f) Klonen der Hybride aus den Fusionsexperimenten,
(g) Screening der geklonten Hybride auf ihre Reaktivität gegenüber gp120,
(h) Vermehrung der Hybride, die positiv reagiert haben,
(i) Screening der Hybride auf solche, die Antikörper bilden, die an einen Kohlenhydratanteil innerhalb des Aminosäurebereichs von etwa Aminosäure 301 bis etwa Aminosäure 358 binden.

9. Verfahren nach Anspruch 8, wobei die nichthumanen Tiere BALB/c-Mäuse sind, und die Myelomzellen SP2/O-Zellen sind.

10. Verfahren nach Anspruch 9, wobei die Antikörper die immunologische Bindungseigenschaften des monoklonalen Antikörpers aufweisen, der durch eine Hybridomzellinie gebildet wird, die bei der American Type Culture Collection unter ATCC No. HB10399 hinterlegt ist.

11. Verfahren nach Anspruch 9, wobei die Antikörper die immunologische Bindungseigenschaften des monoklonalen Antikörpers aufweisen, der von einer Hybridomzellinie gebildet wird, die bei der American Type Culture Collection unter ATCC No. HB10400 hinterlegt ist.

## Revendications (Revendications pour l'(les) Etat(s) contractant(s) suivant(s): AT, BE, CH, LI, DE, DK, FR, GB, GR, IT, NL, SE)

1. Anticorps monoclonal qui se fixe spécifiquement sur un épitope d'une glycoprotéine du virus du syndrome immunodéficitatire acquis humain (VIH), ayant un poids moléculaire d'environ 120 000 daltons (gp120), caractérisé en ce que ledit anticorps reconnaît une portion glucidique de gp120 localisée dans la région comprise entre environ l'acide aminé 301 et environ l'acide aminé 358.

2. Anticorps monoclonal selon la revendication 1, ayant pour caractéristique supplémentaire d'être capable de neutraliser substantiellement le VIH.

3. Anticorps monoclonal ayant les caractéristiques de fixation immunologique de l'anticorps monoclonal produit par une lignée cellulaire d'hybridomes déposée à l'American Culture Type collection, ATCC No. HB10399.

4. Anticorps monoclonal ayant les caractéristiques de fixation immunologique de l'anticorps monoclonal produit par une lignée cellulaire d'hybridomes déposée à l'American Culture Type collection, ATCC No. HB10399.

5. Hybridome capable de produire un anticorps qui reconnaît la portion glucidique de gp120 du VIH, dans laquelle ledit anticorps monoclonal se fixe sur une portion glucidique localisée dans la région comprise entre environ l'acide aminé 301 et environ l'acide aminé 358.

6. Utilisation d'une lignée cellulaire d'hybridomes ATCC No. HB10399 pour la production d'anticorps dirigé contre la portion glucidique de gp120 du VIH.

7. Utilisation d'une lignée cellulaire d'hybridomes ATCC No. HB10400 pour la production d'anticorps dirigé con tre la portion glucidique de gp120 du VIH.

8. Utilisation d'un anticorps monoclonal tel que défini dans l'une quelconque des revendications 1 à 4, dans des dosages immunologiques pour la détection de la présence de VIH.

9. Utilisation d'un hybridome tel que défini dans l'une quelconque des revendications 5 à 7 pour la production d'un anticorps monoclonal.

10. Utilisation d'un anticorps monoclonal tel que défini dans l'une quelconque des revendications 1 à 4, pour la fabrication d'un médicament pour la prévention et le traitement du SIDA.

## Revendications (Revendications pour l'(les) Etat(s) contractant(s) suivant(s): ES)

1. Utilisation d'un anticorps monoclonal qui se fixe spécifiquement sur un épitope d'une glycoprotéine du virus du syndrome immunodéficitatire acquis humain (VIH), ayant un poids moléculaire d'environ 120 000 daltons (gp120), caractérisé en ce que l'anticorps reconnaît une portion glucidique de gp120 localisée dans la région comprise entre environ l'acide aminé 301 et environ l'acide aminé 358, dans des dosages immunologiques, pour la détection de la présence de VIH.

2. Utilisation selon la revendication 1, dans laquelle l'anticorps monoclonal a pour caractéristique supplémentaire d'être capable de neutraliser substantiellement VIH.

3. Utilisation d'un anticorps monoclonal ayant les caractéristiques de fixation immunologique de l'anticorps monoclonal produit par une lignée cellulaire d'hybridomes déposée à l'American Culture Type collection, ATCC No. HB10399, dans des dosages immunologiques pour la détection de la présence de VIH.

4. Utilisation d'un anticorps monoclonal ayant les caractéristiques de fixation immunologiques de l'anticorps monoclonal produit par une lignée cellulaire d'hybridomes déposée à l'American Culture Type collection, ATCC No. HB10399, dans des dosages immunologiques pour la détection de la présence de VIH.

5. Utilisation d'un hybridome capable de produire un anticorps qui reconnaît la portion glucidique de gp120 du VIH, dans laquelle ledit anticorps monoclonal se fixe sur une portion glucidique localisée dans la région comprise entre environ l'acide aminé 301 et environ l'acide aminé 358, dans des dosages immunologiques pour produire un anticorps monoclonal.

6. Utilisation d'une lignée cellulaire d'hybridomes ATCC No. HB10399 pour la production d'anticorps dirigé contre la portion glucidique de gp120 du VIH.

7. Utilisation d'une lignée cellulaire d'hybridome ATCC No. HB10400 pour produire un anticorps dirigé contre la portion glucidique de gp120 du VIH.

8. Procédé de production d'un hybridome capable de produire un anticorps monoclonal qui reconnaît la protion glucidique de gp120 de VIH, dans lequel ledit anticorps monoclonal se fixe sur une portion glucidique localisée dans la région comprise entre environ l'acide aminé 301 et environ l'acide aminé 358, ledit procédé comprenant les étapes consistant à :
(a) imuniser des animaux non humains avec la glycoprotéine gp120 du VIH;
(b) criblage desdits animaux pour la présence d'anticorps dirigé contre gp120;
(c) isolement des cellules de rate des animaux manifestant une réponse;
(d) fusion desdites cellules de rate avec des cellules de myélome;
(e) criblage desdits hybrides sélectionnés pour la production d'anticorps qui se fixent sur l'antigène immunisant;
(f) clonage des hybrides issus des expériences de fusion;
(g) criblage des hybrides clonés pour leur réactivité vis-à-vis de gp120;
(h) amplification des hybrides qui réagissent positivement;
(i) criblage des hybrides pour sélectionner ceux qui produisnt des anticorps qui se fixent sur une portion glucidique localisée dans la région aminoacide comprise entre environ l'acide aminé 301 et environ l'acide aminé 358.

9. Procédé selon la revendication 8, dans lequel lesdits animaux non humains sont des souris BALB/c et lesdites cellules de myélome sont des cellules SP2/O.

10. Procédé selon la revendication 9, dans lequel lesdits anticorps ont les caractéristiques de fixation immunologique de l'anticorps monoclonal produit par une lignée cellulaire d'hybridome déposée à l'American Type Culture collection, ATCC No. HB10399.

11. Procédé selon la revendication 9, dans lequel lesdits anticorps ont les caractéristiques de fixation immunologique de l'anticorps monoclonal produit par une lignée cellulaire d'hybridome déposée à l'American Type Culture collection, ATCC No. HB10400.
